# FASCICULE DE BREVET EUROPEEN

(11) **EP 4 427 780 B1**
(45) Date de publication et mention de la délivrance du brevet: **23.07.2025**
(21) Numéro de dépôt: 24156892.2
(22) Date de dépôt: 09.02.2024
(51) Int. Cl.: A61M 16/00, A61M 16/08, A61M 16/10, A61M 16/12, A61M 16/20, A61M 16/22

(54) **INSTALLATION DE FOURNITURE DE GAZ RESPIRATOIRE EN CIRCUIT FERMÉ**
ATEMGASVERSORGUNGSANLAGE MIT GESCHLOSSENEM KREISLAUF
CLOSED-CIRCUIT BREATHING GAS SUPPLY FACILITY

(30) Priorité: 08.03.2023 FR 2302149
(43) Date de publication de la demande: 11.09.2024
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR); PATEL, Akshar, 75007 Paris (FR)
(74) Mandataire: Air Liquide

(56) Documents cités:
- EP-A1- 3 741 415
- EP-A1- 4 108 282
- WO-A1-2022/079642
- SU-A1- 1 456 161
- US-A1- 2009 095 300
- US-A1- 2016 213 879
- US-A1- 2018 169 369
- US-A1- 2020 023 149
- US-B2- 10 183 184

## Description

La présente invention concerne une installation de fourniture de gaz respiratoire, fonctionnant en circuit fermé, incluant un système de recyclage des gaz expirés par le patient et un système de contrôle de la concentration d'oxygène inhalé, permettant une fourniture d'un mélange gazeux enrichi en oxygène (O₂), typiquement un mélange air/oxygène, à un patient en minimisant la consommation d'oxygène provenant d'une source d'oxygène, telle une bouteille d'oxygène sous pression, alimentant l'installation.

L'administration d'O₂ gazeux permet de corriger une situation hypoxémique chez une personne humaine, i.e. un patient, quel que soit son âge, i.e. nouveaux nés, enfants, adolescents, adultes, personnes âgées ou autres, souffrant une pathologie respiratoire engendrant une saturation d'oxyhémoglobine, mesurée dans le sang, inférieure à une valeur de référence reflétant une saturation « normale », par exemple une saturation inférieure à 90%, par exemple une pathologie respiratoire du type Bronchopneumopathie Chronique Obstructive (BPCO), Syndrome de Détresse Respiratoire Aigüe (SDRA) ou autre.

L'oxygène de qualité médicale est fourni au patient « hypoxique » soit au sein d'un l'hôpital, soit hors hôpital, par exemple dans une unité de secours mobile (SAMU, ambulances...) ou encore au domicile du patient considéré.

Selon le cas, l'oxygène peut provenir soit d'une canalisation de gaz, typiquement d'un réseau de fluides hospitalier, soit d'une bouteille de gaz sous pression contenant par exemple de 400 à 1000 L d'O₂ (vol. gazeux) comprimé jusqu'à 200 bar abs ou plus (mesuré à 1 atm).

Le débit d'oxygène délivré par une bouteille de gaz est réglé selon l'indication visée. Dans les cas les plus aigus, il peut être d'environ 15 L/min pour garantir une haute concentration d'oxygène inhalé, c'est-à-dire proche de 100%, comme par exemple en situation d'urgence lors d'une noyade ou d'une intoxication au monoxyde de carbone (CO). D'autres indications nécessitent un débit plus faible, par exemple de l'ordre de 6 L/min, afin de corriger une hypoxémie plus légère. En règle générale, un débit de 6 L/min équivaut chez un adulte à une concentration en oxygène inhalé de l'ordre de 40% en vol.

Dans tous les cas, l'administration d'oxygène, quel que soit le débit, est de permettre à la saturation en oxyhémoglobine de revenir à la valeur normale, typiquement d'au moins 90% environ.

Pour des raisons d'encombrement et de poids, beaucoup d'unités d'urgence, tel que celles de pompiers, de sauveteurs internationaux ou de militaires, préfèrent utiliser une source d'oxygène gazeux aussi compacte que possible, par exemple une bouteille de type B2, c'est-à-dire une bouteille de 2L de volume interne (équiv. en eau) pouvant contenir de l'oxygène (O₂) comprimé à 200 bar (bouteille pleine), ce qui correspond à un volume de l'ordre de 400L d'oxygène.

Or, on comprend que l'autonomie conférée par une telle bouteille, c'est-à-dire le temps d'utilisation avant qu'elle ne soit vide de gaz, varie en fonction du débit de soutirage, qui lui-même dépend de la gravité de la pathologie à traiter. Ainsi, pour un débit de 6 L/min, l'autonomie est d'environ 1h, alors qu'elle est inférieure à 30 minutes pour un débit de 15 L/min.

Dès lors, l'autonomie peut s'avérer insuffisante, par exemple lors d'un transport de longue durée, typiquement lors d'une évacuation sanitaire d'un pays à un autre, ou depuis une zone reculée, comme lors d'une opération militaire sur théâtre d'opération extérieur, ou encore pendant un secours aux victimes en cas de désastre majeur, tel un tremblement de terre ou autre.

EP 4 108 282 A1, EP 3 741 415 A1, US 2018/169369 A1 et WO 2022/079642 A1 proposent différents types de systèmes de fourniture de gaz respiratoire à un patient.

US-A-2021/0121649 propose un système de fourniture de gaz, i.e. d'oxygène, en circuit fermé composé d'une bouteille d'oxygène comprimé munie d'un détendeur de gaz, d'une tubulure alimentant un réservoir de gaz et un module contenant de la chaux sodée pour purifier les gaz expirés par le patient (i.e. victime) en piégeant le dioxyde de carbone (CO₂) qu'y s'y trouve. Il réinhale ensuite ses propres gaz expirés débarrassés du CO₂. Sachant que près de 95% de l'oxygène inhalé est expiré (i.e. non métabolisé), purifier les gaz expirés permet de considérablement réduire le débit fourni par la source d'oxygène, donc d'augmenter l'autonomie puisque le débit d'oxygène métabolisé est inférieur à 1L/min environ.

Cependant, un tel système n'est pas idéal car il ne permet pas de maîtriser la concentration en oxygène inhalé. En effet, dans ce système, la bouteille d'oxygène délivre un débit fixe supérieur à la consommation métabolique du patient, par exemple à 1L/min environ, ce qui peut conduire des situations hyperoxiques, c'est-à-dire à délivrer une concentration en oxygène trop élevée au patient, i.e. proche de 100%. Il peut alors en résulter des conséquences physiologiques néfastes pour chez certains patient, comme par exemple chez ceux en arrêt cardiaque, qui sont ventilés pendant le massage cardiaque et ce, jusqu'au retour à une circulation sanguine spontanée (ROSC).

Un problème est dès lors de pourvoir éviter ce type de problème en contrôlant la concentration en oxygène inhalé par le patient, lors de l'utilisation d'un système de fourniture d'oxygène en circuit fermé. Autrement dit, l'invention vise à améliorer les systèmes de fourniture d'oxygène en circuit fermé, tel celui décrit par US-A-2021/0121649.

Une solution selon l'invention concerne une installation de fourniture de gaz respiratoire à un utilisateur, i.e. un patient, comprenant :
- une source de gaz pour fournir un gaz respiratoire contenant de l'oxygène,
- une interface respiratoire,
- une ligne principale d'acheminement de gaz reliant fluidiquement la source de gaz à l'interface respiratoire,
- une ligne de récupération de gaz en communication fluidique avec l'interface respiratoire,
- un système de purification de gaz alimenté par la ligne de récupération de gaz,
- une ligne de recyclage de gaz reliant fluidiquement le système de purification de gaz à la ligne principale d'acheminement de gaz,
- et des moyens de pilotage.

De plus, l'installation de fourniture de gaz comprend en outre :
- un capteur d'oxygène agencé pour mesurer la concentration d'oxygène au sein de la ligne principale d'acheminement de gaz et fournir la ou les mesures de concentration d'oxygène aux moyens de pilotage,
- une ligne d'admission reliée fluidiquement à l'atmosphère et par ailleurs à la ligne principale d'acheminement de gaz entre la source de gaz et le capteur d'oxygène,
- un dispositif de sélection de concentration configuré pour permettre à un utilisateur, typiquement un personnel soignant, de sélectionner ou fixer une concentration cible d'O₂ inhalé et de fournir la concentration cible d'O₂ inhalé sélectionnée aux moyens de pilotage, et
- un premier élément proportionnel agencé sur la ligne d'admission et commandé par les moyens de pilotage pour contrôler la circulation d'air dans la ligne d'admission.

Selon le mode de réalisation considéré, l'installation de fourniture de gaz respiratoire de l'invention peut comprendre l'une ou plusieurs des caractéristiques suivantes :
- l'installation est du type à circuit fermé.
- la ligne d'admission comprend une valve unidirectionnelle d'admission.
- les moyens de pilotage sont configurés pour commander le premier élément proportionnel, pour autoriser une circulation d'air dans la ligne d'admission, lorsque les moyens de pilotage déterminent qu'au moins une mesure de concentration d'oxygène mesurée est supérieure à la concentration cible d'O₂ inhalé fixée ou sélectionnée.
- le premier élément proportionnel est configuré pour adopter au moins :
   a) une position de fermeture interdisant toute circulation d'air dans la ligne d'admission, et
   b) une position d'ouverture autorisant une circulation d'air dans la ligne d'admission.
- les moyens de pilotage sont configurés pour commander le passage du premier élément proportionnel de la position de fermeture à la position d'ouverture, ou inversement.
- l'installation comprend en outre une ligne d'échappement en communication fluidique avec la ligne de récupération de gaz et par ailleurs avec l'atmosphère.
- la ligne d'échappement comprend un second élément proportionnel commandé par les moyens de pilotage pour contrôler la circulation de gaz dans la ligne d'échappement.
- la ligne d'échappement comprend une valve unidirectionnelle d'échappement.
- le second élément proportionnel est configuré pour adopter au moins :
   a) une position de fermeture interdisant toute circulation de gaz dans la ligne d'échappement, et
   b) une position d'ouverture autorisant une circulation de gaz dans la ligne d'échappement.
- les moyens de pilotage sont configurés pour commander le passage du second élément proportionnel de la position de fermeture à la position d'ouverture, ou inversement.
- la ligne de recyclage de gaz relie fluidiquement le système de purification de gaz à la ligne principale d'acheminement de gaz, via un réservoir tampon.
- le réservoir tampon est, en outre, relié fluidiquement à la source de gaz.
- les moyens de pilotage comprennent au moins un microprocesseur, c'est-à-dire qu'ils constituent une unité de pilotage à microprocesseur(s).
- le dispositif de sélection de concentration comprend une interface graphique utilisateur (IGU).
- le dispositif de sélection de concentration est intégré à l'installation.
- alternativement, le dispositif de sélection de concentration est indépendant de l'installation. Par exemple, il est un téléphone multifonction (smartphone), une tablette numérique, un ordinateur portable ou autre.
- le dispositif de sélection de concentration coopère avec les moyens de pilotage.
- le dispositif de sélection de concentration communique avec les moyens de pilotage.
- le premier élément proportionnel et/ou le second élément proportionnel comprennent au moins une électrovanne proportionnelle ou au moins un robinet de fourniture de gaz motorisé.
- la source de gaz est configurée pour fournir de l'oxygène pur en tant que gaz respiratoire.
- la source de gaz comprend une bouteille d'oxygène comprimé, c'est-à-dire à une pression d'au moins 100 bar, de préférence au moins 150 bar (bar absolus), i.e. pression mesurée lorsque la bouteille est pleine de gaz.
- l'interface respiratoire est configurée pour administrer le gaz respiratoire à l'utilisateur, lors de chaque phase inspiratoire dudit utilisateur.
- la ligne principale d'acheminement de gaz est configurée pour acheminer le gaz respiratoire de la source de gaz à l'interface respiratoire.
- la source de gaz comprend une bouteille d'oxygène comprimé équipée d'un robinet à détendeur intégré ou RDI, c'est-à-dire comprenant des moyens de détente de gaz agencés dans le robinet.
- le robinet à détendeur intégré ou RDI comprend une sortie en débit et une sortie en pression.
- le robinet à détendeur intégré ou RDI comprend un port de sortie en débit et un port de sortie en pression.
- le port de sortie en débit permet de délivrer différents débits que l'on peut choisir au moyen d'un sélecteur de débit, typiquement des débits compris entre 0 et 15L/min.
- le port de sortie en pression permet de fournir une pression gazeuse fixe, typiquement de l'ordre de 4 bar.
- le robinet à détendeur intégré ou RDI, à savoir le port de sortie en débit ou, selon le cas, le port de sortie en pression du RDI, est relié fluidiquement au réservoir-tampon via une ligne de fourniture de gaz, tel un tuyau flexible ou analogue.
- une ligne de récupération de gaz est configurée pour récupérer et acheminer au moins une partie du mélange gazeux CO₂/O₂ expiré se retrouvant dans l'interface respiratoire, lors de chaque phase expiratoire de l'utilisateur.
- la ligne principale d'acheminement de gaz comprend un (ou des) passage ou conduit de gaz ou analogue.
- le système de purification de gaz est alimenté en mélange gazeux CO₂/O₂ expiré par la ligne de récupération de gaz.
- le système de purification de gaz est configuré pour éliminer au moins une partie du CO₂ contenu dans le mélange gazeux CO₂/O₂ expiré et obtenir un gaz purifié.
- la ligne de recyclage de gaz est configurée pour acheminer au moins une partie du gaz purifié provenant du système de purification de gaz et le réinjecter dans la ligne principale d'acheminement de gaz.
- la ligne de recyclage de gaz comprend un (ou des) passage ou conduit de gaz ou analogue.
- l'interface respiratoire est un masque respiratoire, en particulier un masque facial, i.e. bucco-nasal, couvrant le nez et la bouche de l'utilisateur.
- l'interface respiratoire est un masque respiratoire comprenant un orifice d'entrée d'oxygène pour fournir le gaz riche en oxygène et un orifice de sortie de gaz expiré pour évacuer le mélange gazeux expiré par l'utilisateur, c'est-à-dire les gaz expirés enrichis en CO₂.
- le masque respiratoire comprend un coussin souple venant en contact et assurant une étanchéité fluidique avec le visage de l'utilisateur.
- le masque respiratoire comprend un corps de masque, par exemple en polymère rigide, formant une coque délimitant une enceinte pour le gaz.
- le coussin souple est couplé mécaniquement au corps de masque, de préférence de manière détachable.
- le coussin souple comprend un passage central au sein duquel viennent se loger le nez et la bouche de l'utilisateur, lorsqu'il porte le masque facial.
- l'utilisateur respire le gaz contenu dans l'enceinte, c'est-à-dire inspire et expire le gaz dans l'enceinte du corps de masque.
- l'orifice d'entrée d'oxygène et l'orifice de sortie de gaz expiré sont aménagés dans le corps de masque.
- la ligne principale d'acheminement de gaz et/ou la ligne de récupération de gaz et/ou la ligne de recyclage de gaz sont ou comprennent une ou des conduites de gaz souples, c'est-à-dire des tuyaux flexibles, par exemple en polymère.
- la ligne principale d'acheminement de gaz et/ou la ligne de récupération de gaz viennent se raccorder au corps de masque de manière à être en communication fluidique avec l'orifice d'entrée et l'orifice de sortie de gaz expiré.
- la ligne principale d'acheminement de gaz est raccordée fluidiquement à l'orifice d'entrée de l'interface respiratoire de manière à introduire du gaz respiratoire dans l'interface respiratoire.
- la ligne de récupération de gaz est raccordée fluidiquement à l'orifice de sortie de gaz expiré de l'interface respiratoire de manière à extraire du gaz respiratoire de l'interface respiratoire.
- la ligne de recyclage de gaz alimente le réservoir-tampon avec du gaz purifié étant donné que la ligne de recyclage de gaz est raccordée fluidiquement au réservoir-tampon.
- le gaz purifié acheminé par la ligne de recyclage de gaz contient une proportion (i.e. teneur) d'oxygène inférieure ou égale au gaz provenant de la source de gaz, i.e. d'oxygène pur, et acheminé par la ligne principale d'acheminement de gaz.
- le réservoir-tampon comprend un ballon flexible, un soufflet ou analogue.
- le réservoir-tampon comprend une valve d'échappement permettant d'évacuer à l'atmosphère tout excès de gaz, lorsque le réservoir-tampon est plein de gaz.
- le réservoir-tampon est dimensionné pour contenir de 0.5 à 10 litres de gaz (à l'état non-comprimé).
- le réservoir-tampon est configuré pour permettre d'y réaliser un mélange de gaz à partir de gaz respiratoire.
- le réservoir-tampon est configuré pour alimenter la ligne principale d'acheminement de gaz avec le gaz respiratoire.
- le système de purification de gaz comprend au moins un adsorbant présentant une sélectivité supérieure pour le CO₂ que pour l'O₂ de manière à adsorber au moins une partie du CO₂ contenu dans le mélange gazeux expiré.
- le système de purification de gaz comprend au moins un adsorbant zéolitique.
- le système de purification de gaz comprend au moins un adsorbant choisi parmi les silicates.
- le système de purification de gaz comprend un adsorbeur contenant au moins un adsorbant, de préférence un adsorbant zéolitique ou silicate, ou leurs mélanges.
- l'adsorbeur contiennent au moins un lit d'adsorbant.
- le système de purification de gaz comprend au moins un adsorbant pour éliminer le CO₂ et la vapeur d'eau (H₂O), c'est-à-dire que le CO₂ et la vapeur d'eau sont éliminés sur un même lit d'adsorbant, par exemple un lit de zéolite unique, ou sur des lits successifs d'absorbants différents, par exemple un lit de silicate et un lit de zéolite.
- l'adsorbeur a une forme de cartouche.
- les moyens de pilotage comprennent un microcontrôleur.
- une source de courant électrique (i.e. des moyens d'alimentation électrique) fournit du courant électrique à l'installation, par exemple une liaison au courant du secteur (110/220V) de type cordon électrique et prise de raccordement, et/ou une (ou des) batterie d'alimentation électrique, de préférence rechargeable, et/ou un transformateur de courant.
- la source de courant électrique alimente en courant tous les composants nécessitant de courant pour fonctionner, notamment les moyens de pilotage et/ou le capteur d'oxygène.
- selon un mode de réalisation, un distributeur à électrovanne proportionnelle de dosage est agencé sur la ligne de fourniture de gaz.
- la ligne de fourniture de gaz incluant le distributeur à électrovanne proportionnelle est reliée à la sortie en pression du RDI de la source de gaz.
- le distributeur comprend une ligne interne de dosage en communication fluidique avec la ligne de fourniture de gaz, en particulier avec un flexible de raccordement formant une portion amont de la ligne de fourniture de gaz et avec une ligne d'approvisionnement formant une portion aval de la ligne de fourniture de gaz.
- l'électrovanne proportionnelle de dosage est agencée sur la ligne interne de dosage du distributeur pour contrôler le passage du gaz sous pression (e.g. de l'ordre de 4 bar), typiquement de l'oxygène, au sein de la ligne interne de dosage.
- l'électrovanne proportionnelle de dosage est commandée par les moyens de pilotage, en particulier l'ouverture de l'électrovanne proportionnelle de dosage et/ou le débit fourni par l'électrovanne proportionnelle de dosage.
- l'électrovanne proportionnelle de dosage est naturellement fermée lorsque non-commandée par les moyens de pilotage.
- l'IGU permet de régler ou sélectionner l'ouverture de l'électrovanne proportionnelle de dosage et/ou le débit fourni par l'électrovanne proportionnelle de dosage.

Il est à noter que, dans le cadre de la présente invention, on utilise indifféremment la terminologie « le gaz expiré » ou « les gaz expirés » (i.e. singulier ou pluriel) pour désigner le mélange gazeux rejeté par les poumons du patient, lequel contient de l'O₂, du CO₂ et généralement de la vapeur d'eau, voire d'autres constituants, comme de l'argon ou de l'azote.

L'invention va maintenant être mieux comprise grâce à la description détaillée suivante, faite à titre illustratif mais non limitatif, en référence aux figures annexées parmi lesquelles :
- Fig. 1 schématise un mode de réalisation d'une installation de fourniture de gaz respiratoire à un utilisateur selon la présente invention.
- Fig. 2 schématise une variante du mode de réalisation de l'installation de Fig. 1.

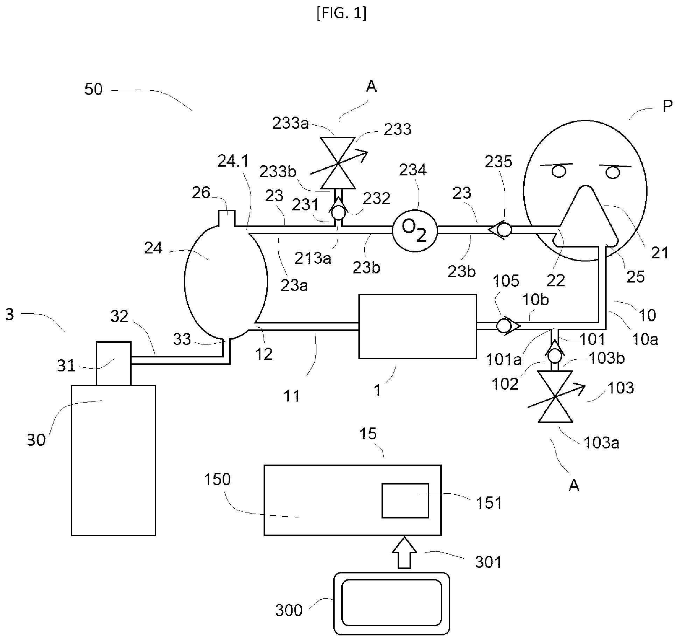

Fig. 1 schématise un mode de réalisation d'une installation de fourniture de gaz respiratoire 50 selon la présente invention incluant un système de recyclage des gaz expirés avec contrôle de la concentration d'oxygène inhalé.

Cette installation 50 comprend une source de gaz 3 pour fournir un gaz respiratoire contenant de l'oxygène ; une ligne principale d'acheminement de gaz 23, aussi appelée circuit patient ; une interface respiratoire 21, tel un masque facial, pour administrer le gaz respiratoire à l'utilisateur ou patient P, lors de ses phases inspiratoires ; et un système cartouche de purification 1 des gaz expirés.

La source de gaz 3 peut être par exemple une bouteille d'oxygène 30 comprimé munie d'un robinet détendeur intégré 31 ou RDI muni de deux ports (non représentés), à savoir un port de sortie en débit permettant de délivrer différents débits que l'on peut choisir au moyen d'un sélecteur de débit, par exemple des débits de 1 à 15L/min et un port de sortie en pression permettant une connexion dite « haute pression », pour fournir une pression gazeuse fixe, typiquement de l'ordre de 4 bar ; ce port de sortie en pression pouvant être aussi utilisé pour relier la source d'O₂ à un ventilateur médical. De préférence, le sélecteur de débit permet de régler le débit d'O₂ délivré par le RDI de manière à fournir un débit faible, à savoir de l'ordre de 1 L/min par exemple, dans le cadre de l'invention.

La ligne principale d'acheminement de gaz 23, i.e. le circuit patient, comprend un tuyau ou conduit flexible qui relie fluidiquement un réservoir-tampon 24 en communication fluidique avec le lumen de la ligne principale d'acheminement de gaz 23, via un port de sortie 24.1 du réservoir-tampon 24, servant de réserve de gaz respiratoire (i.e. d'oxygène ou d'un mélange enrichi en oxygène), à un orifice ou port d'entrée 22 de l'interface respiratoire 21 de manière à alimenter le patient P en gaz respiratoire, typiquement en O₂ ou en un gaz riche en oxygène, comme un mélange air/O₂. Lorsque le patient P respire, le réservoir-tampon 24 qui est rempli de gaz satisfait la demande instantanée en gaz du patient P en lui fournissant du gaz qu'il renferme. Le réservoir tampon 24 a une capacité entre par exemple 0.5 et 10L (équivalent en eau), préférentiellement de l'ordre de 3 L.

Le RDI 31 de la source de gaz 3 est fluidiquement connecté au réservoir-tampon 24 via une ligne de fourniture de gaz 32, par exemple une canule, un tuyau flexible ou un conduit par exemple en silicone, alimentant un port de fourniture de gaz 33 du réservoir tampon 24.

Agencée dans la ligne principale d'acheminement de gaz 23 se trouve une valve unidirectionnelle d'inhalation 235 ne permettant le passage du gaz présent dans la ligne principale d'acheminement de gaz 23 que dans un sens, à savoir ici dans le sens allant du réservoir tampon 24 vers le port d'entrée 22 de l'interface respiratoire 21, en interdisant tout cheminement inverse du gaz.

Par ailleurs, un capteur d'oxygène 234 est également placé dans la ligne principale d'acheminement de gaz 23, permettant de mesurer la concentration d'O₂ présente dans la ligne principale 23. Le capteur d'oxygène 234 est préférentiellement de type rapide, c'est-à-dire ayant une réaction rapide à un changement de concentration en oxygène, par exemple de l'ordre de 1 à 2 secondes. On peut utiliser par exemple le capteur d'oxygène référencé FDO2 disponible auprès de la société Pyroscience GmbH. Le capteur d'oxygène 234 est agencé ici en amont de la valve unidirectionnelle d'inhalation 235.

Une ligne d'admission 231 est reliée fluidiquement à la ligne principale d'acheminement de gaz 23, en amont du capteur d'oxygène 234, c'est-à-dire au niveau d'un site ou embranchement 231a situé entre le réservoir tampon 24 et le capteur d'oxygène 234.

La ligne d'admission 231 comprend une valve unidirectionnelle d'admission 232 et un premier élément proportionnel 233 permettant de contrôler la circulation de gaz, typiquement d'air, dans la ligne d'admission 231.

La valve unidirectionnelle d'admission 232 est configurée pour n'autoriser le passage de gaz, i.e. d'air, que dans le sens allant de l'atmosphère ambiante A à la ligne principale d'acheminement de gaz 23, i.e. interdisant tout cheminement de gaz dans le sens opposé.

Par ailleurs, le premier élément proportionnel 233 comprend un port amont 233a en communication fluidique avec l'atmosphère ambiante A et un port aval 233b en communication fluidique avec la ligne d'admission 231. Pour assurer un contrôle de la circulation de gaz, i.e. d'air ambiant, le premier élément proportionnel 233 est configuré pour adopter plusieurs positions comprenant (au moins) une position fermée et une position ouverte, i.e. partiellement ou totalement ouverte, telle que :
- en position fermée, le port amont 233a est isolé du port aval 233b, c'est-à-dire que la communication fluidique entre eux est interrompue et/ou empêchée ;
- en position ouverte, le port amont 233a communique avec le port aval 233b, c'est-à-dire que la communication fluidique, totale ou partielle (i.e. ouverture proportionnelle), entre eux est possible et/ou rétablie.

Le premier élément proportionnel 233 est par exemple un robinet de fourniture de gaz motorisé, c'est-à-dire à moteur électrique, tel celui référencé USS-MSV00001 disponible auprès de la société US. SOLID, ou tout autre dispositif de contrôle adapté ayant un fonctionnement similaire, par exemple une électrovanne proportionnelle de large diamètre de passage.

Pendant le fonctionnement de l'installation 50, le gaz expiré par le patient P, qui est riche en CO₂ mais contient encore de l'oxygène non-métabolisé, ressort de l'interface respiratoire 21 par un port ou orifice de sortie 25 aménagé dans l'interface respiratoire 21, avant d'être récupéré et convoyé par une ligne de récupération de gaz 10 formant un circuit expiratoire, tel un conduit ou tuyau de gaz flexible, connecté fluidiquement au port de sortie 25 de l'interface respiratoire 21.

Cette ligne de récupération de gaz 10 permet de récupérer et acheminer au moins une partie du mélange gazeux expiré se retrouvant dans l'interface respiratoire 21, lors des phases expiratoires de l'utilisateur P. Ce gaz expiré contient différents composés gazeux, majoritairement de l'O₂ et du dioxyde de carbone (CO₂), mais aussi de la vapeur d'eau (H₂O), et généralement aussi de l'azote (N₂), voire de l'argon (Ar).

Dans tous les cas, le mélange gazeux expiré contient une proportion de CO₂ non nulle puisque résultant de la respiration du patient et des échanges pulmonaires entre O₂ et CO₂, par exemple une proportion de CO₂ de l'ordre de 5 % vol.

Le flux de gaz expiré est acheminé par la ligne de récupération de gaz 10 jusqu'à un système de purification 1 de gaz expiré, telle une (ou des) cartouche d'adsorption, au sein duquel le CO₂ et éventuellement la vapeur d'eau sont retenus ou captés, typiquement adsorbés, par un (ou des) lit de d'adsorbant(s) contenant préférentiellement des particules d'adsorbant(s), par exemple agencées en lit d'adsorbant. Par exemple, on peut utiliser des adsorbants de type zéolite ou autres.

Le gaz purifié produit par le système de purification 1 contient essentiellement de l'oxygène et une proportion faible, voire négligeable, de CO₂ et/ou de vapeur d'eau (i.e. humidité) et éventuellement d'autres composés résiduels sous forme de traces ou d'impuretés inévitables, tel de l'azote et/ou de l'argon.

Autrement dit, le système de purification de gaz 1 comprenant une (ou des) cartouche d'adsorption, qui est alimenté en gaz expiré par la ligne de récupération de gaz 10, permet d'éliminer la majeure partie, préférentiellement la (quasi-)totalité, du CO₂ et de la vapeur d'eau contenue dans le mélange gazeux expiré et d'obtenir ainsi un gaz purifié contenant essentiellement de l'oxygène, voire des impuretés inévitables ou traces de CO₂ ou d'autre(s) composé(s).

Par exemple, une cartouche d'adsorption de ce type comprend au moins un adsorbant présentant une sélectivité supérieure pour le CO₂ que pour l'O₂ de manière à adsorber au moins une partie du CO₂ contenu dans le mélange gazeux expiré et, de préférence, la (quasi-)totalité du CO₂. L'adsorbant peut être de type zéolite ou silicate.

Préférentiellement, l'adsorbant est agencé en un lit d'adsorbant, comme un lit de zéolite, ou en plusieurs lits successifs, comme par exemple un lit de silicate et un lit de zéolite et ce, de manière à pouvoir éliminer non seulement le CO₂ mais aussi au moins une partie de la vapeur d'eau (H₂O), c'est-à-dire que le CO₂ et la vapeur d'eau sont éliminés sur un lit unique d'adsorbant, par exemple un lit de zéolite unique, ou sur des lits successifs d'absorbants différents. Bien entendu, d'autres matériaux adsorbants peuvent convenir.

Avantageusement, une fois saturée de CO₂, voire aussi de vapeur d'eau, la cartouche est remplacée par une cartouche neuve.

Là encore, une valve unidirectionnelle d'expiration 105, agencée dans la ligne de récupération de gaz 10, n'autorise la circulation du gaz que dans le sens allant de l'orifice de sortie 25 à la cartouche de purification de gaz 1, c'est-à-dire interdisant tout cheminement inverse du gaz.

Par ailleurs, est prévue aussi une ligne d'échappement 101 en communication fluidique avec la ligne de récupération de gaz 10, venant se raccorder l'une à l'autre en un site de raccordement ou embranchement 101a.

La ligne d'échappement 101 comprend une valve unidirectionnelle d'échappement 102 et un second élément proportionnel 103 similaire au premier élément proportionnel 233. Le second élément proportionnel 103 comprend, lui aussi, un port amont 103a en communication fluidique avec l'atmosphère ambiante A et un port aval 103b en communication fluidique avec la ligne d'échappement 101, et son fonctionnement est identique à celui du premier élément proportionnel 233, à savoir qu'il est configuré pour adopter plusieurs positions comprenant (au moins) une position fermée et une position ouverte, i.e. partiellement ou totalement ouverte, afin d'interdire ou d'autoriser, respectivement, tout passage de gaz en direction de l'atmosphère, c'est-à-dire tout échappement de gaz vers l'atmosphère ambiante A. La valve unidirectionnelle d'échappement 102 ne permet donc le passage du gaz que dans le sens allant de la ligne de récupération de gaz 10 vers l'atmosphère ambiante A.

L'installation 50 comprend aussi, agencée en aval du système de purification de gaz 1, une ligne de recyclage de gaz 11 reliée fluidiquement, d'une part, au système de purification de gaz 1 et, d'autre part, au réservoir-tampon 24, de manière à recueillir le gaz purifié sortant du système de purification de gaz 1 et à l'acheminer jusqu'au réservoir 24, dans lequel il pénètre via le port d'entrée 12 auquel est raccordée fluidiquement la ligne de recyclage de gaz 11.

Le port d'entrée 12 et le port de fourniture de gaz 33 sont places à proximité l'un et de l'autre afin d'améliorer l'homogénéisation des gaz dans le réservoir-tampon 24.

Une valve d'échappement 26 permettant d'évacuer à l'atmosphère ambiante tout excès de gaz au sein du réservoir-tampon 24, lorsqu'il est plein.

Afin d'assurer un recyclage des gaz expirés avec contrôle de la concentration d'oxygène inhalé, l'installation 50 comprend par ailleurs des moyens de pilotage 15 comprenant une carte de commande électronique 150 et une unité de contrôle 151 à microprocesseur, typiquement un microcontrôleur ou analogue.

Les moyens de pilotage 15 permettent de contrôler ou commander tous les éléments électromécaniques de l'installation 50 de l'invention. Plus précisément, la carte de commande 150 intègre préférentiellement l'unité de contrôle 151 et est configurée pour commander et par ailleurs analyser les signaux provenant des différents composants de l'installation 50, tels que vannes, capteurs...

L'alimentation électrique de l'installation 50 de l'invention est assurée par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant.

Les moyens de pilotage 15 et d'autres composants de l'installation 50 sont agencés dans une carcasse ou boitier externe rigide (non montré), par exemple en polymère.

Comme déjà dit, le système de purification de gaz 1 de l'installation 50 permet de purifier et ensuite recycler les gaz expirés en les débarrassant du CO₂ et préférentiellement de la vapeur d'eau (H₂O) qu'ils contiennent, au lieu de les rejeter à l'atmosphère et ainsi gaspiller l'oxygène qui s'y trouve encore.

En effet, récupérer la majeure partie de l'O₂ contenu dans les gaz expirés par le patient P et la recycler après purification, permet d'éviter son gaspillage, et donc ainsi de limiter le débit d'oxygène fourni par la source d'O₂ 3.

Ainsi, par exemple, si l'on considère que la consommation métabolique d'O₂ d'un être humain est de l'ordre de 0.5 L/min, qui correspond à une ventilation minute de 10 L/min, i.e. 10L de gaz (100% d'O₂) inhalé par le patient pendant une minute, une quantité d'environ 9.5 L d'O₂ est expirée, le reste, i.e. environ 0.5 L/min étant formé de CO₂ et de vapeur d'eau. L'installation 50 de l'invention permet donc de récupérer environ 9.5L d'O₂. En réglant le RDI 31 de la bouteille 30 à un débit de 1 L/min et en recyclant l'O₂ en le mélangeant avec l'O₂ provenant de la bouteille 30, le débit d'O₂ récupéré excède la ventilation minute du patient.

On considère, d'abord, que les moyens de pilotage 15 commandent les premier et second éléments proportionnels 233, 103 en position fermée de manière à isoler les lignes d'admission et d'échappement 231, 101 de l'atmosphère ambiante A.

Par ailleurs, la source de gaz 3 est réglée de manière à fournir un débit constant de 1 L/min d'oxygène, alors que le patient P a une consommation métabolique d'O₂ de l'ordre de 0.5 L/min et que le réservoir tampon 24 est rempli d'O₂ (teneur 100%), provenant de la source de gaz 3.

Lors d'une phase inspiratoire, c'est-à-dire lorsque le patient P réalise une inhalation de gaz, le gaz inhalé sort du réservoir tampon 24 et chemine dans la ligne principale d'acheminement de gaz 23 jusqu'au port d'entrée 22 de l'interface respiratoire 21.

A l'inverse, lors d'une phase expiratoire (suivant la phase inspiratoire), le gaz expiré par le patient P ressort de l'interface respiratoire 21 par le port de sortie 25 et est récupéré dans la ligne de récupération de gaz 10 pour ensuite être convoyé au système de purification 1 des gaz expirés, où le CO₂ est éliminé (et préférentiellement la vapeur d'eau, voire d'autres composés présents, à l'exception de l'oxygène). Le gaz purifié obtenu est convoyé par la ligne de recyclage de gaz 11 et remplit à nouveau le réservoir tampon 24 via son port d'entrée 12.

Afin de mieux comprendre l'invention, on considère que le réservoir tampon 24 contient ici un volume de 3L d'O₂ pur (i.e. 100% vol.), et que les poumons du patient P ont un volume total de l'ordre de 5 L et qu'ils contiennent de l'air (i.e. mélange de 21% d'O₂ et d'azote ; l'argon et les autres gaz éventuels sous forme de trace sont négligés), avant la première inhalation d'oxygène. Dès lors, l'ensemble a un volume total de l'ordre de 8 L et la concentration initiale en O₂ est de l'ordre de 50% et est homogène entre le réservoir tampon 24 et les poumons du patient P.

Le gaz purifié, après son passage dans le système de purification 1, est formé essentiellement d'oxygène et d'azote mais il ne contient plus de CO₂, sachant que le volume de CO₂ expiré par minute est toujours inférieur ou égal à la consommation métabolique en O₂ d'un patient, aussi appelé « quotient respiratoire ».

La source de gaz 3 étant réglée à un débit constant supérieur à la consommation métabolique du patient P (i.e. ici 1 L/min d'apport pour une consommation métabolique de 0.5 L/min), l'apport en O₂ par la source de gaz 3 est alors en excès et le réservoir tampon 24 atteint sa capacité maximale pendant l'expiration du patient P. Le gaz purifié pénétrant dans le réservoir tampon 24, via son port d'entrée 12, va s'homogénéiser rapidement avec l'O₂ provenant de la source de gaz 3 via le port de fourniture de gaz 33.

Dès lors, l'excédent en volume évacué à l'atmosphère par la valve d'échappement 26, lorsque le réservoir-tampon 24 est plein, contient de l'azote et de l'O₂. Au fil du temps, c'est-à-dire des phases d'inhalation et d'expiration successives, le volume global d'azote (N₂) restant dans l'ensemble patient P/installation 50 a tendance à diminuer, étant donné que l'azote est progressivement remplacé par de l'O₂ fourni par la source de gaz 3, c'est-à-dire d'oxygène pur. Il s'ensuit qu'après un temps donné, la concentration d'O₂ atteint environ 100% et reste à ce niveau (présence éventuelle de traces d'azote résiduel ou d'impuretés inévitables).

Or, comme déjà expliqué, une telle concentration élevée d'oxygène n'est pas souhaitable au plan médical car pouvant engendrer des situations hyperoxiques chez certains patients.

Pour y remédier, selon l'invention, les moyens de pilotage 15 de l'installation de l'invention 50 sont configurés pour permettre au personnel soignant de sélectionner une concentration cible d'O₂ inhalé, via des moyens ou un dispositif de sélection de concentration 300, typiquement une concentration cible d'O₂ inhalé inférieure ou égale à 100% vol.

Selon le mode de réalisation, le dispositif de sélection de concentration 300 peut être intégré à l'installation, tel une IGU ou interface graphique utilisateur comprenant des touches de sélection ou analogue et un afficheur, ou être indépendant de celle-ci, par exemple le dispositif de sélection de concentration 300 peut être un téléphone multifonction (i.e. smartphone), une tablette numérique, un ordinateur portable ou un autre dispositif communiquant 301, à distance ou non, avec les moyens de pilotage 15, via un protocole de communication de type Bluetooth^{®} ou autre.

Dans tous les cas, l'installation de l'invention 50 coopère avec le dispositif de sélection de concentration 300 qui permet au personnel soignant de choisir ou fixer une concentration cible d'O₂ inhalé et de fournir la concentration cible d'O₂ inhalé sélectionnée ou fixée aux moyens de pilotage 15 de l'installation de l'invention 50 qui agissent en réponse à la réception de cette concentration cible d'O₂ inhalé sélectionnée, comme expliqué ci-après.

On prévoit donc des moyens de réception, tel qu'un receveur Bluetooth^{®}, intégrés ou coopérant avec les moyens de pilotage pour recevoir la concentration cible d'O₂ inhalé sélectionnée ou fixée via le dispositif de sélection de concentration 300.

Si le circuit fermé formé notamment par la ligne principale d'acheminement de gaz 23, la ligne de récupération de gaz 10 et la ligne de recyclage de gaz 11, permet de limiter la consommation de la source d'O₂ 3, et donc d'augmenter l'autonomie de prise en charge du patient P, il est cliniquement désirable de permettre à l'utilisateur, i.e. le personnel soignant, de pouvoir sélectionner une concentration en oxygène inhalé désirée afin de répondre aux besoins physiologiques spécifiques du patient P considéré, typiquement une concentration cible d'O₂ inhalé inférieure ou égale à 100% vol.

Ainsi, si l'utilisateur désire une concentration en O₂ inhalé égale à 40% par exemple, il la fixe ou la sélectionne ou la choisit via le dispositif de sélection de concentration 300 et cette teneur ou concentration cible d'O₂ inhalé désirée est alors fournie aux moyens de pilotage 15 sous forme de signal ou autre.

Lorsque les moyens de pilotage 15 reçoivent le signal correspondant à ce choix de concentration désirée, i.e. ici de 40%, ils le traitent et par ailleurs interagissent avec le capteur d'oxygène 234, de façon périodique, par exemple toutes les 0.5 secondes, pour opérer une mesure de la concentration d'O₂ au sein de la ligne principale d'acheminement de gaz 23.

La ou les mesures de concentration d'O₂ (i.e. valeur ou signal) sont fournies par le capteur d'oxygène 234 aux moyens de pilotage 15 qui les traitent, en particulier les comparent à la concentration désirée, i.e. 40%.

Une concentration mesurée supérieure au choix de concentration réalisé (i.e. 40%), par exemple égale à 100%, est une indication pour les moyens de pilotage 15 que la concentration actuelle mesurée est supérieure à la concentration cible souhaitée, donc est trop élevée, et doit donc être ajustée pour éviter ou minimiser les problèmes susmentionnés.

En réponse, les moyens de pilotage 15 commandent le premier élément proportionnel 233 pour le passer dans une position au moins partiellement ouverte, ce qui créé une communication fluidique entre l'atmosphère ambiante A et la ligne d'admission 231, comme explicité précédemment, et donc permettre un apport d'air ambiant.

Lors de l'inhalation par le patient P, le gaz issu du réservoir tampon 24 transitant dans la portion amont 23a de la ligne principale d'acheminement de gaz 23, contenant de l'O₂ à 100%, va alors être additionné (i.e. dilué par) d'air transitant dans la ligne d'admission 231, lors de l'inspiration du patient P, pour former ainsi un mélange gazeux air/O₂ de concentration en O₂ donnée, i.e. inférieure à 100% (du fait de la dilution opérée), dans une portion aval 23b de la ligne principale d'acheminement de gaz 23, située en aval du site d'embranchement 213a.

La concentration en O₂ obtenue dans le mélange gazeux air/O₂ est alors mesurée par le capteur d'oxygène 234. Elle dépend du rapport entre le volume de gaz provenant du réservoir tampon 24 et le volume de gaz (i.e. air ambiant) provenant de la ligne d'admission 231, donc aussi du niveau d'ouverture du premier élément proportionnel 233.

Les moyens de pilotage 15 peuvent alors déterminer, au moyen d'un ou plusieurs algorithmes mémorisés, quel niveau d'ouverture donner au premier élément proportionnel 233, par exemple en analysant l'écart entre la mesure de la concentration en O₂ par le capteur d'oxygène 234 et la concentration d'oxygène cible sélectionnée ou fixée par l'utilisateur, et en prenant en compte le volume du réservoir tampon 24.

Autrement dit, le niveau d'ouverture du premier élément proportionnel 233 est proportionnel notamment à la différence entre la concentration en O₂ fournie par le capteur d'oxygène 234 et à la concentration d'oxygène cible désirée.

Lors de l'expiration du patient P, sachant que le second élément proportionnel est toujours en position fermée, la (quasi)totalité des gaz expirés par le patient P ressort de l'interface respiratoire 21, est acheminée par la ligne de récupération de gaz 10, et est traitée par le système de purification 1 pour y retenir le CO₂ (et l'eau). Le gaz purifié va aller remplir à nouveau le réservoir tampon 24, sachant qu'il contient alors une concentration ou teneur en O₂ inférieure a 100%, par exemple de l'ordre de 60%.

Du fait du mélange du gaz purifié au contenu gazeux du réservoir tampon 24 et à l'oxygène provenant de la source d'oxygène 3, la concentration résiduelle dans le réservoir tampon 24 deviendra inférieure à 100%, par exemple de l'ordre de 80%.

Dès lors, pendant l'inhalation suivante par le patient P, le gaz transitant dans la portion amont 23a de la ligne principale d'acheminement de gaz 23 a une concentration en O₂ inférieure à 100%, par exemple de l'ordre de 80%.

Avantageusement, les moyens de pilotage 15 anticipent cette baisse de concentration en O₂ par le biais d'un ou plusieurs algorithmes spécifiques et ajustent, en conséquence, le niveau d'ouverture du premier élément proportionnel 233 afin que l'apport en air circulant dans la ligne d'admission 231, se mélangeant au gaz circulant dans la portion amont 23a de la ligne principale d'acheminement de gaz 23, présente une concentration moyenne dans la portion aval 23b de la ligne principale d'acheminement de gaz 23, mesurée par le capteur d'O₂, se rapprochant encore plus de la concentration d'O₂ cible souhaitée.

On comprend que du fait de la succession d'inhalations et d'expirations de gaz par le patient P au fil du temps, en mesurant la concentration d'O₂ dans la portion aval 23b de la ligne principale d'acheminement de gaz 23 et en contrôlant précisément le niveau d'ouverture du premier élément proportionnel 233, les moyens de pilotage 15 peuvent progressivement faire converger la concentration en O₂ inhalé réelle (i.e. mesurée) vers la concentration d'O₂ cible souhaitée par l'utilisateur.

Une fois la concentration d'O₂ cible atteinte, les moyens de pilotage 15 entrent, préférentiellement, en mode dit « de maintien » en pilotant précisément, c'est-à-dire en fermant ou ouvrant légèrement, le premier élément proportionnel 233 afin de maintenir la concentration en O₂ inhalé par le patient P égal ou (très) proche de la concentration en O₂ cible souhaitée par le personnel soignant.

De façon inverse, il peut survenir des situations où le patient P inhale une concentration donnée, par exemple 40%, mais que son état se détériore au point que le personnel soignant désire augmenter le niveau de concentration en O₂ inhalée par le patient P, par exemple à 90% (au lieu de 40% par exemple).

Dans ce cas, les moyens de pilotage 15 peuvent commander une fermeture du premier élément proportionnel 233 pour empêcher toute entrée d'air et donc laisser la concentration en O₂ présente dans la ligne principale d'acheminement de gaz 23 atteindre progressivement la valeur souhaitée (i.e. 90%) grâce à l'apport d'O₂ par la source gaz 3 qui est en excès (i.e. très supérieur à) par rapport à la consommation métabolique du patient P, pour ensuite rentrer en phase de maintien en fermant ou ouvrant légèrement le premier élément proportionnel 233 afin de maintenir la concentration en O₂ inhalé par le patient P proche de la concentration en O₂ cible désirée, comme expliqué ci-avant.

Cependant, le temps requis pour atteindre un tel état peut être parfois trop long, voire même cliniquement inacceptable car non compatible avec l'état du patient à traiter. Dans ce cas, les moyens de pilotage 15 sont configurés pour commander l'installation pour accélérer l'augmentation de concentration en oxygène dans le flux de gaz fourni au patient P via l'interface respiratoire 21.

Pour ce faire, les moyens de pilotage 15 sont configurés pour déterminer la concentration d'O₂ régnant dans la ligne principale d'acheminement de gaz 23, grâce aux mesures opérées par le capteur O₂ 234, en particulier déterminée si cette concentration d'O₂ mesurée est inférieure à la concentration d'O₂ cible souhaitée.

Si tel est le cas, les moyens de pilotage 15 commandent le premier élément proportionnel 233 en position fermée pour interdire ou interrompre, au sein du conduit 231, toute circulation d'air provenant de l'atmosphère ambiante, et en parallèle commandent le second élément proportionnel 103 en position ouverte (i.e. partiellement ou totalement ouverte) et créé ainsi une communication fluidique entre ligne d'échappement 101 et l'atmosphère ambiante A, comme explicité précédemment.

Ainsi, lors de l'expiration du patient P, le gaz circulant dans la portion amont 10a de la ligne de récupération de gaz 10, ayant une concentration en O₂ de 40% de par exemple (donc contenant environ 60% d'azote et éventuellement d'autres impuretés inévitables, tel de l'argon ou de la vapeur d'eau) se divise, à l'embranchement 101a, en deux flux gazeux.

Plus précisément, une partie du gaz (i.e. premier flux) circulant dans la portion amont 10a de la ligne de récupération de gaz 10 transite dans la ligne d'échappement 101 pour s'échapper, i.e. être rejetée, à l'atmosphère ambiante A, alors que le reste du gaz (i.e. second flux) emprunte la portion aval 10b de la ligne de récupération de gaz 10 pour être acheminé jusqu'au système de purification 1 des gaz expirés, où le CO₂ est éliminé. Le gaz purifié qui en ressort par la ligne de recyclage de gaz 11 va remplir à nouveau le réservoir tampon 24 via son port d'entrée 12.

Le rapport volumique (i.e. quantités respectives) entre le flux gaz empruntant la ligne d'échappement 101 et le flux gaz empruntant la portion aval 10b de la ligne de récupération de gaz 10 dépend du niveau d'ouverture du second élément proportionnel 103. Les moyens de pilotage 15 sont configurés pour déterminer, au moyen d'un ou plusieurs algorithmes spécifiques, par exemple en analysant l'écart entre la mesure de la concentration en O₂ par le capteur O₂ 234 et la concentration en O₂ cible, quel niveau d'ouverture donner au second élément proportionnel 103.

Dans tous les cas, le volume de gaz acheminé au réservoir tampon 24 est inférieur au volume de gaz ayant circulé dans la portion amont 10a de la ligne de récupération de gaz 10.

Schématiquement, si 1L de gaz expiré a transité dans la ligne d'échappement 101 pour être déchargé à l'atmosphère A, l'ensemble patient P et installation 50 est assimilable à un volume total de 7L à une concentration en O₂ de 40%, complémenté par 1L d'O₂ a 100% issu de la source de gaz 3. La concentration en O₂ résultante rapportée au volume de 8L est alors de l'ordre de 47.5%. Bien entendu, le complément en O₂ pur issu de la source de gaz 3 ne peut être délivré instantanément du fait du réglage initial de 1L/min, ce qui implique que le réservoir tampon 24 se vide progressivement au gré des inhalations et expirations successives du patient P. Pendant celles-ci, en fonction de la mesure de concentration en O₂ dans la ligne la ligne principale d'acheminement de gaz 23, les moyens de pilotage 15, déterminent le degré d'ouverture à conférer au second élément proportionnel 103 afin qu'une partie des gaz expirés par le patient P soient évacués à l'atmosphère A par la ligne d'échappement 101.

Dans le cadre d'une augmentation de la concentration en O₂ cible, il peut être demandé à l'utilisateur, e.g. au personnel soignant, par le biais de l'interface de commande 300, telle une IGU, d'augmenter de façon manuelle le débit issu de la source de gaz 3, par exemple de le fixer à 6L/min, de manière à garantir que le réservoir tampon 24 ne se vide pas complètement. Le second élément proportionnel 103 permet de « purger » l'azote plus rapidement que la seule valve d'échappement 26 du réservoir tampon 24, ce qui permet d'atteindre plus rapidement la nouvelle concentration en O₂ cible, lorsque celle-ci est supérieure à la concentration régnant dans la ligne principale d'acheminement de gaz 23.

Une fois la concentration en O₂ cible atteinte, les moyens de pilotage 15 entrent en mode de maintien en fermant le second élément proportionnel 103 et en fermant ou ouvrant légèrement le premier élément proportionnel 233 afin de maintenir la concentration en O₂ inhalé par le patient P à un niveau proche de la concentration en O₂ cible.

On comprend donc que l'installation 50 selon l'invention permet de réguler avec précision la teneur en oxygène envoyée au patient P en fonction d'une (ou plusieurs) teneur ou concentration d'oxygène cible fixée par le personnel soignant en fonction des besoins du patient P considéré et ce, notamment en jouant sur l'apport d'air ambiant ou sur l'évacuation de gaz du circuit vers l'atmosphère ambiante A.

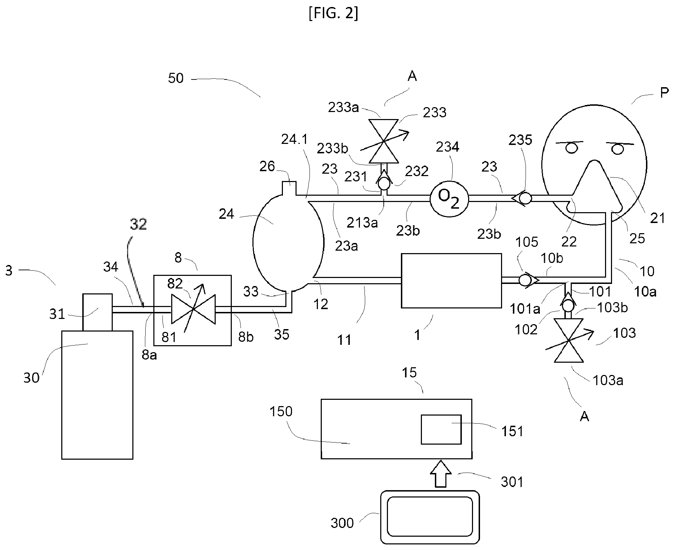

Une variante de l'installation 50 selon l'invention est présentée en Fig. 2. L'architecture globale de l'installation 50 de Fig. 2 est identique à celle de Fig. 1 et ne sera pas détaillée pour éviter des répétitions inutiles.

Cette variante de l'installation 50 permet de réduire encore, voire d'optimiser, le temps nécessaire à l'obtention d'une concentration cible en O₂ inhalé réglée par l'utilisateur lorsque celle-ci est supérieure à la concentration en O₂ inhalé.

Pour ce faire, on utilise préférentiellement le port « haute pression » du RDI 31 de la source de gaz 3 qui contient de l'oxygène comprimé, que l'on connecte, via un flexible de raccordement 34 formant une portion amont de la ligne de fourniture de gaz 32, au port amont 8a d'un distributeur 8 qui se trouve alors alimenté par de l'oxygène à 4 bar fourni par le flexible de raccordement 34.

Le distributeur 8 comprend une électrovanne proportionnelle de dosage 82 contrôlée par les moyens de pilotage 15, laquelle est agencée sur une ligne interne de dosage 81 qui est en communication fluidique avec le flexible de raccordement 34 formant la portion amont de la ligne de fourniture de gaz 32.

L'électrovanne proportionnelle de dosage 82 est naturellement fermée lorsque non commandée par les moyens de pilotage 15 de manière à ce qu'aucun débit d'O₂ provenant du RDI 31 de la source de gaz 3 ne circule dans la ligne de dosage 81, et ne pénètre dans le réservoir tampon 24 par le biais du port d'approvisionnement en O₂ 33, via une ligne d'approvisionnement 35 formant une portion aval de la ligne de fourniture de gaz 32, reliée au port aval 8b du distributeur 8. La ligne d'approvisionnement 35 est par exemple un tuyau flexible.

L'électrovanne proportionnelle de dosage 82 peut par exemple celle référencée « VSO LowPro » disponible auprès de la société Parker ou toute autre électrovanne équivalente ou analogue.

Lors du fonctionnement de l'installation 50, les moyens de pilotage 15 déterminent un niveau d'ouverture de l'électrovanne proportionnelle de dosage 82 de manière à ce que celle-ci délivre un débit de l'ordre de 1L/min, appelé débit de base, comme décrit ci-avant en référence à Fig. 1. Connaissant la pression de détente de 4 bar, on peut déterminer la position de fonctionnement de l'électrovanne proportionnelle de dosage 82. Bien entendu, des éléments supplémentaires, tels qu'un capteur de débit, de pression... peuvent être utilisés pour réguler un tel débit de base.

L'IGU 300 communiquant avec les moyens de pilotage 15 peut par ailleurs permettre le réglage du débit de base par le personnel soignant, se rapprochant de préférence du métabolisme supposé du patient, par exemple un débit de 0.5 L/min, 1L/min ou plus. Dans ce cas, l'électrovanne proportionnelle de dosage 82 délivre le débit de base qui circule dans la ligne d'approvisionnement 35 et remplit le réservoir tampon 24.

L'intérêt d'un tel distributeur 8 réside notamment dans les phases transitoires, notamment lors d'un réglage d'une concentration cible en O₂ supérieure à la concentration en O₂ inhalé par le patient. En effet, dans ce cas, les moyens de pilotage 15 peuvent ouvrir pleinement le second élément proportionnel 103 afin que la majorité, voire la totalité des gaz expirés circulant dans la ligne de récupération des gaz 10, s'échappe vers l'atmosphère ambiante A, via la ligne d'échappement 101, afin de permettre une évacuation rapide de l'azote présent dans l'installation 50.

Dans le même temps, les moyens de pilotage 15 peuvent contrôler l'électrovanne proportionnelle de dosage 82 de manière à augmenter son débit, par exemple à 6L/min ou plus, afin de remplir le réservoir tampon 24 et éviter que celui-ci ne se vide.

Une fois que la concentration cible en O₂ inhalé est atteinte, les moyens de pilotage 15 peuvent contrôler l'électrovanne proportionnelle de dosage 82 de manière à ce que celle-ci délivre à nouveau un débit de base préréglé. Une tel fonctionnement permet alors d'améliorer (i.e. réduire) le temps de convergence vers une nouvelle concentration cible lorsque celle-ci est supérieure à la concentration en O₂ inhalé, d'améliorer le débit d'O₂ en automatisant une tâche devant être réalisée par l'utilisateur et, in fine, de renforcer la sécurité globale de l'installation 50.

L'installation de fourniture 50 de gaz respiratoire selon la présente invention permet de contrôler la concentration en O₂ inhalé par un patient P tout en limitant fortement la consommation en O₂ provenant de la source de gaz 3, augmentant ainsi son autonomie.

## Revendications

1. Installation de fourniture (50) de gaz respiratoire à un utilisateur (P) comprenant :
- une source de gaz (3) pour fournir un gaz respiratoire contenant de l'oxygène,
- une interface respiratoire (21),
- une ligne principale d'acheminement de gaz (23) reliant fluidiquement la source de gaz (3) à l'interface respiratoire (21),
- une ligne de récupération de gaz (10) en communication fluidique avec l'interface respiratoire (21),
- un système de purification de gaz (1) alimenté par la ligne de récupération de gaz (10),
- une ligne de recyclage de gaz (11) reliant fluidiquement le système de purification de gaz (1) à la ligne principale d'acheminement de gaz (23),
- et des moyens de pilotage (15),
**caractérisée en ce qu'**elle comprend en outre :
- un capteur d'oxygène (234) agencé pour mesurer la concentration d'oxygène au sein de la ligne principale d'acheminement de gaz (23) et fournir la ou les mesures de concentration d'oxygène aux moyens de pilotage (15),
- une ligne d'admission (231) reliée fluidiquement à l'atmosphère et par ailleurs à la ligne principale d'acheminement de gaz (23) entre la source de gaz (3) et le capteur d'oxygène (234),
- un dispositif de sélection de concentration (300) configuré pour permettre à un utilisateur de sélectionner ou fixer une concentration cible d'O₂ inhalé et de fournir la concentration cible d'O₂ inhalé sélectionnée aux moyens de pilotage (15), et
- un premier élément proportionnel (233), agencé sur la ligne d'admission (231), commandé par les moyens de pilotage (15) pour contrôler la circulation d'air dans la ligne d'admission (231).

2. Installation selon la revendication 1, **caractérisée en ce que** la ligne d'admission (231) comprend une valve unidirectionnelle d'admission (232).

3. Installation selon la revendication 1, **caractérisée en ce que** les moyens de pilotage (15) sont configurés pour commander le premier élément proportionnel (233), pour autoriser une circulation d'air dans la ligne d'admission (231), lorsque les moyens de pilotage (15) déterminent qu'au moins une mesure de concentration d'oxygène mesurée est supérieure à la concentration cible d'O₂ inhalé fixée ou sélectionnée.

4. Installation selon la revendication 1 ou 3, **caractérisée en ce que** le premier élément proportionnel (233) est configuré pour adopter au moins :
- une position de fermeture interdisant toute circulation d'air dans la ligne d'admission (231), et
- une position d'ouverture autorisant une circulation d'air dans la ligne d'admission (231),
lesdits moyens de pilotage (15) étant configurés pour commander le passage du premier élément proportionnel (233) de la position de fermeture à la position d'ouverture, ou inversement.

5. Installation selon la revendication 1, **caractérisée en ce qu'**elle comprend en outre une ligne d'échappement (101) en communication fluidique avec la ligne de récupération de gaz (10) et par ailleurs avec l'atmosphère, ladite ligne d'échappement (101) comprenant un second élément proportionnel (103) commandé par les moyens de pilotage (15) pour contrôler la circulation de gaz dans la ligne d'échappement (101).

6. Installation selon la revendication 5, **caractérisée en ce que** la ligne d'échappement (101) comprend une valve unidirectionnelle d'échappement (102).

7. Installation selon la revendication 5, **caractérisée en ce que** le second élément proportionnel (103) est configuré pour adopter au moins :
- une position de fermeture interdisant toute circulation de gaz dans la ligne d'échappement (101), et
- une position d'ouverture autorisant une circulation de gaz dans la ligne d'échappement (101),
les moyens de pilotage (15) étant configurés pour commander le passage du second élément proportionnel (103) de la position de fermeture à la position d'ouverture, ou inversement.

8. Installation selon la revendication 1, **caractérisée en ce que** la ligne de recyclage de gaz (11) relie fluidiquement le système de purification de gaz (1) à la ligne principale d'acheminement de gaz (23), via un réservoir tampon (24), le réservoir tampon (24) étant, en outre, relié fluidiquement à la source de gaz (3).

9. Installation selon la revendication 1, **caractérisée en ce que** les moyens de pilotage (15) comprennent au moins un microprocesseur.

10. Installation selon les revendications 1 et 5, **caractérisée en ce que** le premier élément proportionnel (233) et/ou le second élément proportionnel (103) comprennent au moins une électrovanne proportionnelle ou au moins un robinet de fourniture de gaz motorisé.

11. Installation selon la revendication 1, **caractérisée en ce que** le dispositif de sélection de concentration (300) comprend une interface graphique utilisateur (IGU)

12. Installation selon la revendication 1, **caractérisée en ce que** la source de gaz (3) est configurée pour fournir de l'oxygène pur en tant que gaz respiratoire.

13. Installation selon la revendication 1, **caractérisée en ce que** l'interface respiratoire (21) est configurée pour administrer le gaz respiratoire à l'utilisateur.

14. Installation selon la revendication 1, **caractérisée en ce que** le système de purification de gaz (1) est configuré pour éliminer au moins une partie du CO₂ contenu dans le mélange gazeux CO₂/O₂ expiré par l'utilisateur dans l'interface respiratoire (21), et obtenir un gaz purifié.

15. Installation selon les revendications 1 et 14, **caractérisée en ce que** la ligne de récupération de gaz (10) est configurée pour récupérer et acheminer au moins une partie du mélange gazeux CO₂/O₂ expiré se retrouvant dans l'interface respiratoire (21).

## Patentansprüche

1. Anlage zur Bereitstellung (50) von Atemgas für einen Benutzer (P), umfassend:
- eine Gasquelle (3), um ein Sauerstoff enthaltendes Atemgas bereitzustellen,
- eine Atemschnittstelle (21),
- eine Gastransporthauptleitung (23), welche die Gasquelle (3) fluidisch mit der Atemschnittstelle (21) verbindet,
- eine Gasrückgewinnungsleitung (10), die in Fluidverbindung mit der Atemschnittstelle (21) steht,
- ein Gasreinigungssystem (1), das von der Gasrückgewinnungsleitung (10) versorgt wird,
- eine Gasrückführungsleitung (11), die das Gasreinigungssystem (1) fluidisch mit der Gastransporthauptleitung (23) verbindet,
- und Ansteuerungsmittel (15),
**dadurch gekennzeichnet, dass** sie ferner umfasst:
- einen Sauerstoffsensor (234), der dazu ausgebildet ist, die Sauerstoffkonzentration in der Gastransporthauptleitung (23) zu messen und den Ansteuerungsmitteln (15) die Sauerstoffkonzentrationsmessung(en) bereitzustellen,
- eine Einlassleitung (231), die fluidisch mit der Atmosphäre und im Übrigen mit der Gastransporthauptleitung (23) zwischen der Gasquelle (3) und dem Sauerstoffsensor (234) verbunden ist,
- eine Konzentrationsauswahlvorrichtung (300), die dazu ausgestaltet ist, einem Benutzer zu ermöglichen, eine Zielkonzentration des eingeatmeten O₂ auszuwählen oder festzulegen und den Ansteuerungsmitteln (15) die Zielkonzentration des eingeatmeten O₂ bereitzustellen, und
- ein an der Einlassleitung (231) angeordnetes erstes Proportionalelement (233), das von den Ansteuerungsmitteln (15) gesteuert wird, um die Luftzirkulation in der Einlassleitung (231) zu regeln.

2. Anlage nach Anspruch 1, **dadurch gekennzeichnet, dass** die Einlassleitung (231) ein unidirektionales Einlassventil (232) umfasst.

3. Anlage nach Anspruch 1, **dadurch gekennzeichnet, dass** die Ansteuerungsmittel (15) dazu ausgestaltet sind, das erste Proportionalelement (233) zu steuern, um eine Luftzirkulation in der Einlassleitung (231) zuzulassen, wenn die Ansteuerungsmittel (15) feststellen, dass mindestens eine gemessene Sauerstoffkonzentrationsmessung über der festgelegten oder ausgewählten Zielkonzentration des eingeatmeten O₂ liegt.

4. Anlage nach Anspruch 1 oder 3, **dadurch gekennzeichnet, dass** das erste Proportionalelement (233) dazu ausgestaltet ist, mindestens einzunehmen:
- eine Verschlussstellung, die jede Luftzirkulation in der Einlassleitung (231) unterbindet, und
- eine Öffnungsstellung, die eine Luftzirkulation in der Einlassleitung (231) zulässt,
wobei die Ansteuerungsmittel (15) dazu ausgestaltet sind, den Übergang des ersten Proportionalelements (233) aus der Verschlussstellung in die Öffnungsstellung oder umgekehrt zu steuern.

5. Anlage nach Anspruch 1, **dadurch gekennzeichnet, dass** sie ferner eine Auslassleitung (101) umfasst, die in Fluidverbindung mit der Gasrückgewinnungsleitung (10) und im Übrigen mit der Atmosphäre steht, wobei die Auslassleitung (101) ein zweites Proportionalelement (103) umfasst, das von den Ansteuerungsmitteln (15) gesteuert wird, um die Gaszirkulation in der Auslassleitung (101) zu regeln.

6. Anlage nach Anspruch 5, **dadurch gekennzeichnet, dass** die Auslassleitung (101) ein unidirektionales Auslassventil (102) umfasst.

7. Anlage nach Anspruch 5, **dadurch gekennzeichnet, dass** das zweite Proportionalelement (103) dazu ausgestaltet ist, mindestens einzunehmen:
- eine Verschlussstellung, die jede Gaszirkulation in der Auslassleitung (101) unterbindet, und
- eine Öffnungsstellung, die eine Gaszirkulation in der Auslassleitung (101) zulässt,
wobei die Ansteuerungsmittel (15) dazu ausgestaltet sind, den Übergang des zweiten Proportionalelements (103) aus der Verschlussstellung in die Öffnungsstellung oder umgekehrt zu steuern.

8. Anlage nach Anspruch 1, **dadurch gekennzeichnet, dass** die Gasrückführungsleitung (11) das Gasreinigungssystem (1) mit der Gastransporthauptleitung (23) über einen Pufferbehälter (24) fluidisch verbindet, wobei der Pufferbehälter (24) ferner mit der Gasquelle (3) fluidisch verbunden ist.

9. Anlage nach Anspruch 1, **dadurch gekennzeichnet, dass** die Ansteuerungsmittel (15) mindestens einen Mikroprozessor umfassen.

10. Anlage nach den Ansprüchen 1 und 5, **dadurch gekennzeichnet, dass** das erste Proportionalelement (233) und/oder das zweite Proportionalelement (103) mindestens ein Proportionalmagnetventil oder mindestens einen motorisierten Gasbereitstellungshahn umfassen.

11. Anlage nach Anspruch 1, **dadurch gekennzeichnet, dass** die Konzentrationsauswahlvorrichtung (300) eine grafische Benutzerschnittstelle (IGU) umfasst.

12. Anlage nach Anspruch 1, **dadurch gekennzeichnet, dass** die Gasquelle (3) dazu ausgestaltet ist, reinen Sauerstoff als Atemgas bereitzustellen.

13. Anlage nach Anspruch 1, **dadurch gekennzeichnet, dass** die Atemschnittstelle (21) dazu ausgestaltet ist, dem Benutzer das Atemgas zu verabreichen.

14. Anlage nach Anspruch 1, **dadurch gekennzeichnet, dass** das Gasreinigungssystem (1) dazu ausgestaltet ist, mindestens einen Teil des CO₂, das in dem von dem Benutzer ausgeatmeten CO₂/O₂-Gemisch in der Atemschnittstelle (21) enthalten ist, zu entfernen und ein gereinigtes Gas zu erhalten.

15. Anlage nach den Ansprüchen 1 und 14, **dadurch gekennzeichnet, dass** die Gasrückgewinnungsleitung (10) dazu ausgestaltet ist, mindestens einen Teil des ausgeatmeten CO₂/O₂-Gemisches, das sich in der Atemschnittstelle (21) befindet, zurückzugewinnen und zu transportieren.

## Claims

1. Installation (50) for supplying respiratory gas to a user (P), comprising:
- a gas source (3) for supplying an oxygen-containing respiratory gas,
- a respiratory interface (21),
- a main gas delivery line (23) fluidically connecting the gas source (3) to the respiratory interface (21),
- a gas recovery line (10) in fluidic communication with the respiratory interface (21),
- a gas purification system (1) fed by the gas recovery line (10),
- a gas recycling line (11) fluidically connecting the gas purification system (1) to the main gas delivery line (23),
- and operating means (15),
**characterized in that** it additionally comprises:
- an oxygen sensor (234) arranged to measure the oxygen concentration within the main gas delivery line (23) and to supply the oxygen concentration measurement(s) to the operating means (15),
- an intake line (231) fluidically connected to the atmosphere and also to the main gas delivery line (23) between the gas source (3) and the oxygen sensor (234),
- a concentration selection device (300) configured to allow a user to select or set a target concentration of inhaled O₂ and to supply the selected target concentration of inhaled O₂ to the operating means (15), and
- a first proportional element (233), arranged on the intake line (231), controlled by the operating means (15) to control the circulation of air in the intake line (231).

2. Installation according to Claim 1, **characterized in that** the intake line (231) comprises a one-way intake valve (232).

3. Installation according to Claim 1, **characterized in that** the operating means (15) are configured to control the first proportional element (233), so as to allow a circulation of air in the intake line (231) when the operating means (15) determine that at least one measured oxygen concentration measurement is greater than the set or selected target concentration of inhaled O₂.

4. Installation according to Claim 1 or 3, **characterized in that** the first proportional element (233) is configured to adopt at least:
- a closed position prohibiting any circulation of air in the intake line (231), and
- an open position allowing air to circulate in the intake line (231),
said operating means (15) being configured to control the passage of the first proportional element (233) from the closed position to the open position, or vice versa.

5. Installation according to Claim 1, **characterized in that** it additionally comprises an exhaust line (101) in fluidic communication with the gas recovery line (10) and also with the atmosphere, said exhaust line (101) comprising a second proportional element (103) controlled by the operating means (15) to control the circulation of gas in the exhaust line (101).

6. Installation according to Claim 5, **characterized in that** the exhaust line (101) comprises a one-way exhaust valve (102).

7. Installation according to Claim 5, **characterized in that** the second proportional element (103) is configured to adopt at least:
- a closed position prohibiting any circulation of gas in the exhaust line (101), and
- an open position allowing gas to circulate in the exhaust line (101),
the operating means (15) being configured to control the passage of the second proportional element (103) from the closed position to the open position, or vice versa.

8. Installation according to Claim 1, **characterized in that** the gas recycling line (11) fluidically connects the gas purification system (1) to the main gas delivery line (23) via a buffer tank (24), the buffer tank (24) additionally being fluidically connected to the gas source (3).

9. Installation according to Claim 1, **characterized in that** the operating means (15) comprise at least one microprocessor.

10. Installation according to Claims 1 and 5, **characterized in that** the first proportional element (233) and/or the second proportional element (103) comprise at least one proportional solenoid valve or at least one motorized gas-supply valve.

11. Installation according to Claim 1, **characterized in that** the concentration selection device (300) comprises a graphical user interface (GUI).

12. Installation according to Claim 1, **characterized in that** the gas source (3) is configured to supply pure oxygen as respiratory gas.

13. Installation according to Claim 1, **characterized in that** the respiratory interface (21) is configured to administer the respiratory gas to the user.

14. Installation according to Claim 1, **characterized in that** the gas purification system (1) is configured to remove at least some of the CO₂ contained in the CO₂/O₂ gas mixture exhaled by the user in the respiratory interface (21) and to obtain a purified gas.

15. Installation according to Claims 1 and 14, **characterized in that** the gas recovery line (10) is configured to recover and deliver at least some of the exhaled CO₂/O₂ gas mixture found in the respiratory interface (21).
